# EUROPEAN PATENT APPLICATION

(11) **EP 4 257 125 A1**
(43) Date of publication of application: **11.10.2023**
(21) Application number: 21900978.4
(22) Date of filing: 01.12.2021
(51) Int. Cl.: A61K 9/51, A61K 9/00, A61K 35/32, A61P 19/00, A61P 19/02, A61P 29/00, A61P 25/28, A61M 31/00

(54) **METHOD FOR FABRICATING MICROROBOT FOR DELIVERY OF CELL THERAPY PRODUCT AND MICROROBOT ACCORDING THERETO**

(30) Priority: 01.12.2020 KR 20200165388
(71) Applicant: Daegu Gyeongbuk Institute Of Science and Technology, Daegu 42988 (KR); The Catholic University Of Korea Industry-Academic Cooperation Foundation, Seocho-gu Seoul 06591 (KR)
(72) Inventor: CHOI, Hong Soo, Dalseong-gun Daegu 42951 (KR); JEON, Sung Woong, Daejeon 34134 (KR); KIM, Sung Won, Seoul 06001 (KR); PARK, Sun Hwa, Seoul 08794 (KR); KIM, Jin Young, Dalseong-gun Daegu 43016 (KR)
(74) Representative: Haseltine Lake Kempner LLP
(86) International application number: PCT/KR2021/017928
(87) International publication number: WO 2022/119290

(57) **Abstract**

Disclosed are a method for fabricating a microrobot for delivery of a cell therapy product and a microrobot for delivery of a cell therapy product according thereto. A method for fabricating a microrobot for delivery of a cell therapy product is configured to comprise the steps of: coating a magnetic material with a coating material capable of promoting adhesion, proliferation, and differentiation of stem cells; and internalizing or attaching the coated magnetic material into stem cells. The microrobot for delivery of a cell therapy product, fabricated by the fabrication method, is configured to comprise: a stem cell; a magnetic material attached to the surface of the stem cell or injected into the stem cell and enabling migration in response to an external magnetic field; and a coating material applied to the surface of the magnetic material and promoting attachment, proliferation, and differentiation of the stem cell.

## Description

### Technical Field

The following embodiments relate to a method for fabricating a microrobot for delivery of a cell therapy product and a microrobot according thereto.

### Background Art

Stem cells are undifferentiated cells that are less developed, and refer to cells with the ability to differentiate into specific tissue cells. Stem cell therapy using these stem cells is a treatment method that is in the spotlight as a regenerative medicine for the treatment of incurable diseases.

For example, neural stem cells (NSCs) may be used for the treatment of neurodegenerative disorders such as Alzheimer's disease. In addition, stem cells that may be used for the treatment of various diseases such as childhood developmental diseases, metabolic diseases, and cancer have been developed. In particular, recently, it has been reported to develop human nasal inferior turbinate-derived stem cells (hNTSCs) that can be applied to various diseases such as cartilage, bone-related diseases, rheumatoid arthritis, degenerative nervous system diseases, and dementia.

In general, a microrobot is used to transfer the stem cells to an area in need of treatment. The microrobot includes a magnetic body to be movable by an external magnetic field. Treatment is performed by culturing the stem cells in the microrobot including the magnetic body, transferring the microrobot to an affected area, and releasing the stem cells.

However, conventional microrobots for transferring stem cells have cylindrical and hexagonal shapes, and are not equipped with a sufficient number of stem cells by culturing stem cells in a 2D manner.

In addition, in the case of stem cell therapy using the microrobot, there is a disadvantage in that it is difficult to transfer an accurate amount of stem cells to an accurate location when a region requiring treatment is a deep region in the body or a risky region during injection.

In particular, for the treatment of brain nervous system diseases, it is very important to efficiently deliver a therapy product to the brain nervous system. However, since the brain nervous system tissue is surrounded by a hard skull and a blood brain barrier (BBB), the delivery efficiency of therapeutic drugs is very low. In addition, in the case of intravenous or arterial administration, which is a conventional general drug administration method, there is a problem in that a larger amount of drug than a drug required for the affected area is required and side effects according thereto are accompanied.

The aforementioned background art is included or obtained by the present inventors in the process of deriving the disclosure of the present disclosure, and cannot necessarily be known art disclosed to the general public prior to the present application.

### Disclosure of the Invention

### Technical Goals

An aspect of an embodiment is to provide a method for fabricating a microrobot injected into the nasal cavity to directly transfer a cell therapy product to the brain and a microrobot for delivery of a cell therapy product according thereto.

However, technical goals to be achieved are not limited to those described above, and other goals not mentioned above are clearly understood by one of ordinary skill in the art from the following description.

### Technical Solutions

A method for fabricating a microrobot for delivery of a cell therapy product and a microrobot for delivery of a cell therapy product according thereto according to an embodiment are described.

A method for fabricating a microrobot for delivery of a cell therapy product is configured to include coating a magnetic material with a coating material capable of promoting attachment, proliferation, and differentiation of stem cells, and internalizing or attaching the coated magnetic material into stem cells.

According to an embodiment, a single stem cell and the coated magnetic material may become one microrobot.

According to an embodiment, the method may further include culturing a plurality of stem cells in a spheroid shape, and the internalizing or attaching of the coated magnetic material may include internalizing or attaching the coated magnetic material into the stem cells cultured in the spheroid shape.

According to an embodiment, the culturing of the plurality of stem cells in the spheroid shape and the internalizing or attaching of the coated magnetic material into the stem cells may include using at least one of a hanging-drop culture method or a U-shaped 96-well plate culture method.

According to an embodiment, the stem cells may be inferior turbinate-derived stem cells.

According to an embodiment, the inferior turbinate-derived stem cells may be differentiated into one of chondrocytes, bone cells, adipocytes, mucosal differentiation, and nerve cells.

A microrobot for delivery of a cell therapy product is configured to include stem cells, a magnetic material attached to surfaces of the stem cells or injected into the stem cells to enable migration in response to an external magnetic field, and a coating material coated on a surface of the magnetic material and promoting attachment, proliferation, and differentiation of the stem cells.

According to an embodiment, a single stem cell and the magnetic material coated with the coating material may configure one microrobot.

According to an embodiment, a plurality of stem cells cultured in a spheroid shape and the magnetic material coated with the coating material may configure one microrobot.

According to an embodiment, the microrobot may be injected by any one of a first pathway in which an injection device penetrates into the nasal cavity, and the microrobot is injected into the brain along the nasal cavity, and a second pathway in which the injection device penetrates into the brain through the nasal cavity, and the microrobot is injected directly into the brain.

According to an embodiment, the injection pathway of the microrobot may further include a pathway in which the microrobot may also be injected into affected areas with bone or cartilage disease, rheumatoid arthritis, degenerative nervous system disease, brain nervous system disease, and dementia disease.

According to an embodiment, the stem cells may be able to be rotated and translated by an external magnetic field inside an organ by the magnetic material.

According to an embodiment, the stem cells may be inferior turbinate-derived stem cells.

According to an embodiment, the inferior turbinate-derived stem cells may be differentiated into one of chondrocytes, bone cells, adipocytes, mucosal differentiation, and nerve cells.

### Effects

As described above, according to the embodiments, the method for fabricating the microrobot for delivery of the cell therapy product and the microrobot according thereto enable non-invasive treatment by fabricating the microrobot injected into the nasal cavity to directly transfer the cell therapy product to the brain.

The effects of the method for fabricating the microrobot for delivery of the cell therapy product and the microrobot according thereto according to an embodiment are not limited to the effects described above, and other effects not mentioned will be clearly understood by one of ordinary skill in the art from the following description.

### Brief Description of Drawings

FIG. 1 is a flowchart illustrating a method for fabricating a microrobot for delivery of a cell therapy product according to an embodiment.
FIG. 2 is a flowchart illustrating a method for fabricating a microrobot for delivery of a cell therapy product according to another embodiment.
FIG. 3 is a diagram illustrating a method of coating a magnetic material with a coating material in FIG. 1.
FIG. 4 is a diagram illustrating a method of internalizing or attaching a magnetic material into stem cells in FIG. 1.
FIG. 5 is a diagram illustrating a method of culturing a plurality of stem cells in a spheroid shape in FIG. 2.
FIG. 6 is a diagram illustrating a method of internalizing or attaching a magnetic material into a spheroid-shaped stem cell in FIG. 2.
FIG. 7 is a diagram illustrating a first pathway of injecting a microrobot for delivery of a cell therapy product into a patient's body according to an embodiment.
FIG. 8 is a diagram illustrating a second pathway of injecting a microrobot for delivery of a cell therapy product into a patient's body according to an embodiment.

The accompanying drawings of this specification exemplify preferred embodiments of the present disclosure, the technical spirit of the present disclosure will be more understood together with the detailed description of the present disclosure, and thus it will be understood that the present disclosure is not limited to only the contents illustrated in the accompanying drawings.

### Best Mode for Carrying Out the Invention

Hereinafter, embodiments are described in detail with reference to the accompanying drawings. However, since various modifications may be made to the embodiments, the scope of rights of the present application is not limited or restricted by these embodiments. It should be understood that all modifications, equivalents and substitutes for the embodiments are included in the scope of rights of the present application.

The terms used in the embodiments are used for the purpose of description only, and should not be construed to be limited. The singular expression includes the plural expression unless the context clearly dictates otherwise. In the present specification, it should be understood that term "including" or "having" indicates that a feature, a number, a step, an operation, a component, a part, or the combination thereof described in the specification is present, but does not exclude a possibility of presence or addition of one or more other features, numbers, steps, operations, components, parts, or combinations thereof, in advance.

Unless otherwise defined, all terms used herein including technological or scientific terms have the same meanings as those generally understood by a person with ordinary skill in the art to which embodiments pertain. Terms which are defined in a generally used dictionary should be interpreted to have the same meaning as the meaning in the context of the related art, and are not interpreted as ideal or excessively formal meanings unless otherwise defined in the present application.

In addition, in the description with reference to the accompanying drawings, like components designate like reference numerals regardless of reference numerals and a duplicated description thereof will be omitted. In describing the embodiments, a detailed description of related known technologies will be omitted if it is determined that they unnecessarily make the gist of the embodiments unclear.

In addition, in describing the components of the embodiments of the present disclosure, terms including first, second, A, B, (a), (b), and the like may be used. These terms are just intended to distinguish the components from other components, and the terms do not limit the nature, sequence, or order of the components. When it is disclosed that any component is "connected", "coupled", or "linked" to other components, it should be understood that the component may be directly connected or linked to other components, but another component may be "connected", "coupled", or "linked" between the respective components.

Components included in any one embodiment and components having a common function are described using the same names in other embodiments. Unless otherwise stated, descriptions described in any one embodiment may also be applied to other embodiments, and detailed descriptions in the overlapping range will be omitted.

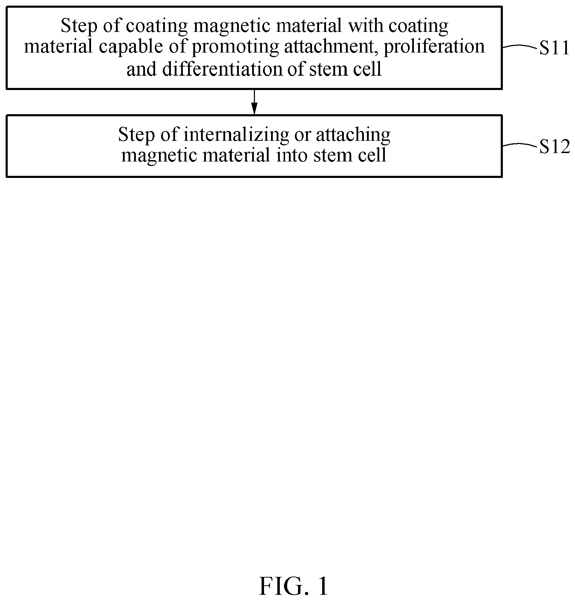

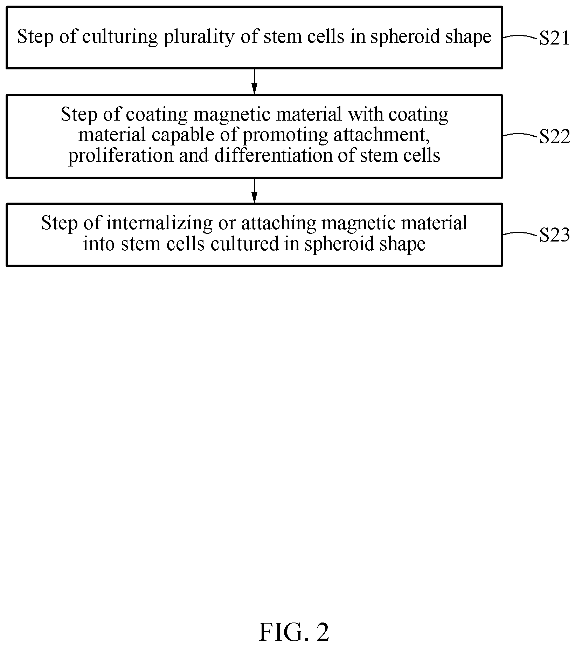

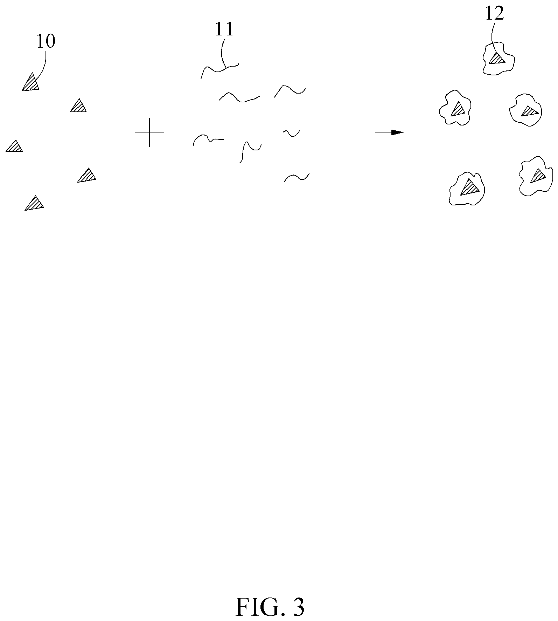

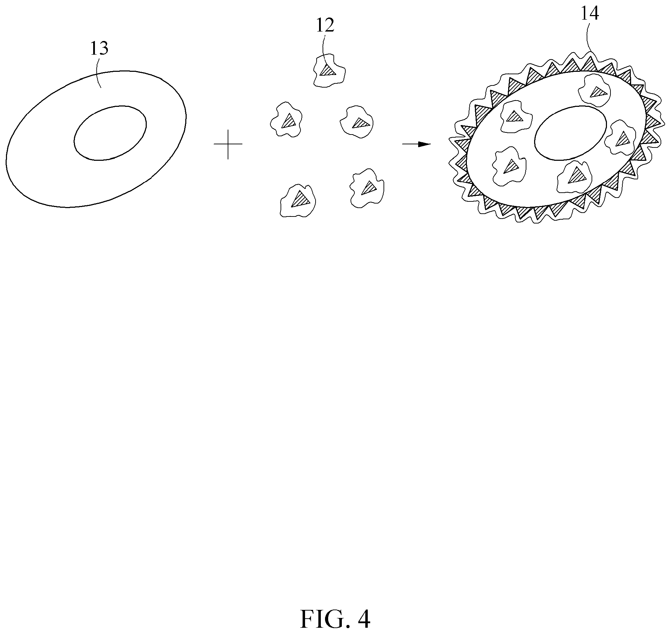

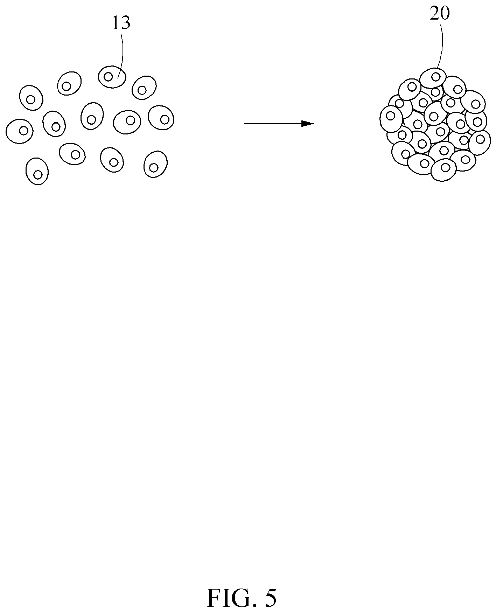

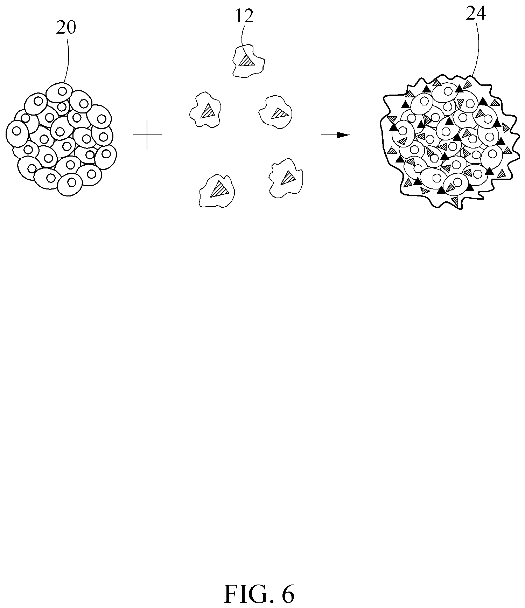

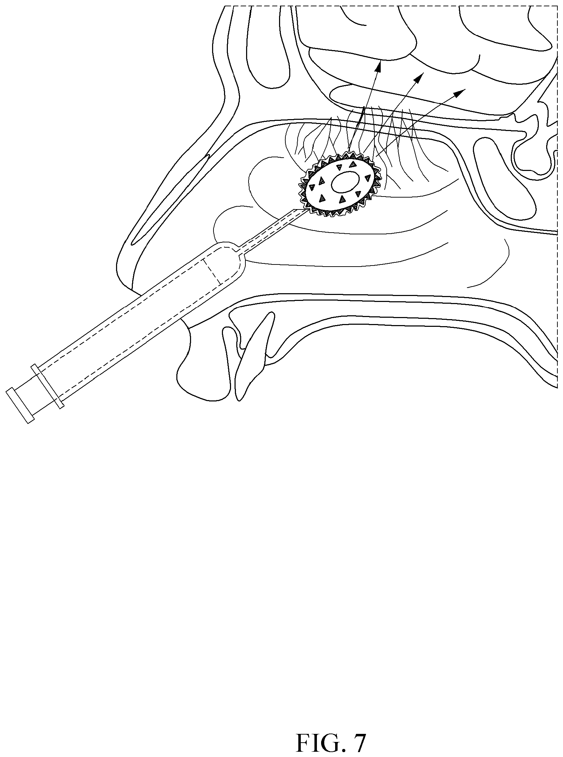

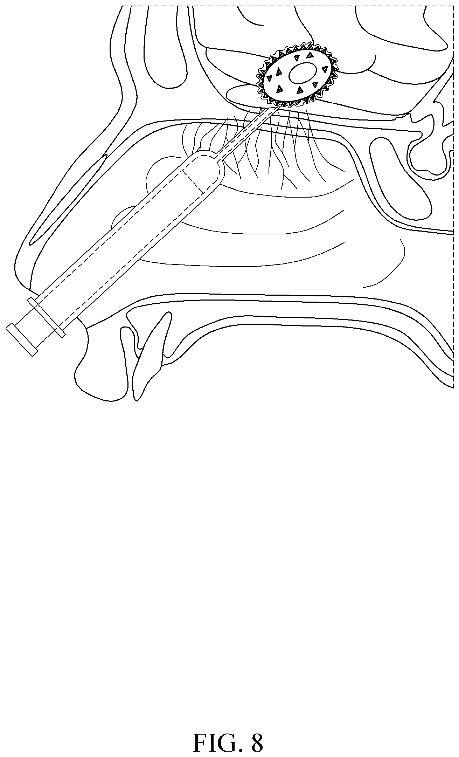

Referring to the drawings, a method for fabricating a microrobot for delivery of a cell therapy product and microrobots 14 and 24 for delivery of the cell therapy product according thereto are described. For reference, FIG. 1 is a flowchart illustrating a method for fabricating a microrobot for delivery of a cell therapy product according to an embodiment, FIG. 2 is a flowchart illustrating a method for fabricating a microrobot for delivery of a cell therapy product according to another embodiment, FIG. 3 is a diagram illustrating a method of coating a magnetic material 10 with a coating material 11 in FIG. 1, FIG. 4 is a diagram illustrating a method of internalizing or attaching a magnetic material 12 into stem cells 13 in FIG. 1, FIG. 5 is a diagram illustrating a method of culturing a plurality of stem cells 13 in a spheroid-shaped stem cell 20 in FIG. 2, FIG. 6 is a diagram illustrating a method of internalizing or attaching a magnetic material 12 into a spheroid-shaped stem cell 20 in FIG. 2, FIG. 7 is a diagram illustrating a first pathway 3 of injecting microrobots 14 and 24 for delivery of a cell therapy product into a patient's body according to an embodiment, and FIG. 8 is a diagram illustrating a second pathway 4 of injecting the microrobots 14 and 24 for delivery of the cell therapy product into a patient's body according to an embodiment.

According to the method for fabricating the microrobot for delivery of the cell therapy product, the microrobots 14 and 24 for delivery of the cell therapy product may be fabricated to be movable in the body by an external magnetic field and transfer stem cells directly to the brain by bypassing a blood brain barrier through a minimal/non-invasive pathway. As a drug delivery structure, the microrobot refers to a structure in size capable of moving in a patient's body and may also be replaced by and fabricated into a nano-sized nanorobot.

Referring to FIG. 1, the method for fabricating the microrobot 14 for delivery of the cell therapy product may be configured to include coating the magnetic material 10 with the coating material 11 capable of promoting attachment, proliferation, and differentiation of the stem cells 13, and internalizing or attaching the coated magnetic material 12 into the stem cells 13.

Here, the stem cells 13 are inferior turbinate-derived stem cells. The inferior turbinate-derived stem cells are obtained by isolating mesenchymal stem cells from human nasal inferior turbinate tissue, and have various differentiation abilities, and thus may be used as therapy products for various diseases. For example, the inferior turbinate-derived stem cells may be differentiated into one of chondrocytes, osteocytes, adipocytes, mucosal differentiation, and nerve cells.

In addition, in the internalizing or attaching of the coated magnetic material 12 into the stem cells 13, the internalizing means a process in which cells absorb and discharge particles such as molecules, viruses, particles, and microorganisms and direct the particles to specific organs in the cytoplasm. Here, a state in which the particles are completely surrounded by the cell membrane and located inside the cell is referred to as the "internalized state," and a state in which the particles are only partially surrounded by the cell membrane and located on the cell surface is referred to as the "attached state."

Referring to FIG. 2, the method for fabricating the microrobot 24 for delivery for the cell therapy product may further include culturing a plurality of stem cells in a spheroid shape. Here, the spheroid refers to a cell aggregate in which multiple single cells gather to form a 3D sphere. Since cells and tissues in the body have a feature of growth, differentiation, and development while interacting in a complex 3D structure, the spheroid has an advantage of having functions equivalent to the body *in vivo.*

Referring to FIGS. 3 and 4, a process for fabricating the microrobot 14 for delivery of the cell therapy product in which a single stem cell 13 and the coated magnetic material 12 configure one microrobot 14 for delivery of the cell therapy product is described.

Referring to FIG. 3, the coating of the magnetic material 10 with the coating material 11 capable of promoting attachment, proliferation, and differentiation of the stem cells 13 may include preparing the magnetic material 10 and the coating material 11 and coating the surfaces of the plurality of magnetic materials 10 with the coating material 11.

Here, the magnetic material 10 may include nano-sized magnetic nanoparticles, and as the magnetic material 10, for example, biocompatible Fe₃O₄ may be used. In addition, various materials having biocompatibility and magnetism may be used.

Here, the coating material 11 may be a polymer material. For example, as the coating material 11, poly-L-lysin may be used as a cationic polymer. The poly-L-lysin has amino groups to have a hydrophilic electropositive property. Here, the positive surface is bound with a negative part of the cell membrane and thus has an effect to increase cell adhesion. In addition, the poly-L-lysin gives an effect of promoting the proliferation and differentiation of the stem cells 13 in the affected area after implantation of the microrobot 14 for delivery of the cell therapy product.

A material other than the poly-L-lysin may be used as the coating material 11 so long as the material has cations in the molecule but is not limited thereto.

Referring to FIG. 4, the internalizing or attaching of the coated magnetic material 12 into the stem cells 13 may include preparing the single stem cell 13 and the magnetic material 12 obtained by coating the magnetic material 10 with the coating material 11 and internalizing or attaching the coated magnetic material 12 into the single stem cell 13.

The coated magnetic material 12 may be internalized into the single stem cell 13 or attached to the surface of the single stem cell 13. The shape in which the single stem cell 13 and the coated magnetic material 12 are attached is simplified and is not limited to the drawings.

The position and direction of the microrobot 14 for delivery of the cell therapy product obtained in this step may be controlled by an external magnetic field system, and the microrobot 14 may be rotated and translated by a rotational magnetic field and may be efficiently transferred in various physiological environments.

Through this process, it is possible to fabricate the microrobot 14 for delivery of the cell therapy product in which the single stem cell 13 and the coated magnetic material 12 configure one microrobot.

Referring to FIGS. 5 and 6, a process for fabricating the microrobot 24 for delivery of the cell therapy product in which a spheroid-shaped stem cell 20 and the coated magnetic material 12 configure one microrobot 24 for delivery of the cell therapy product is described.

Referring to FIG. 5, the plurality of stem cells 13 may be cultured into the spheroid-shaped stem cell 20. In order to culture the plurality of stem cells 13 in a spheroid shape, at least one of a hanging-drop culture method and a U-shaped 96-well plate culture method may be used. For example, after a cell culture solution is applied to a Petri dish, the stem cells 13 may be seeded, and the spheroid-shaped stem cell 20 may be cultured by a 3D cell culture method.

In addition, in the same manner as in FIG. 3, the magnetic material 10 and the coating material 11 may be prepared and the coating material 11 may be coated on the surfaces of the plurality of magnetic materials 10.

Referring to FIG. 6, the internalizing or attaching of the magnetic material into the stem cells cultured in the spheroid shape may include preparing the spheroid-shaped stem cell 20 obtained in FIG. 5 and the magnetic material 12 obtained by coating the magnetic material 10 with the coating material 11 and internalizing or attaching the coated magnetic material 12 into the spheroid-shaped stem cell 20.

The coated magnetic material 12 may be internalized into each stem cell 13 or attached to the surface of the spheroid-shaped stem cell 20. The shape in which the spheroid-shaped stem cell 20 and the coated magnetic material 12 are attached is simplified and is not limited to the drawings.

Through this process, it is possible to fabricate the microrobot 24 for delivery of the cell therapy product in which the spheroid-shaped stem cell 20 and the coated magnetic material 12 configure one microrobot.

The microrobots 14 and 24 for delivery of the cell therapy product fabricated by the above method may be able to be rotated and translated inside blood vessels by an external magnetic field. The microrobots 14 and 24 for delivery of the cell therapy product may be rotated and translated to enable precise position control in physiological environments and to efficiently deliver the stem cells 13 to the affected area.

Here, the external magnetic field may be generated by a magnetic field generating device (not illustrated). The magnetic field generating device may generate a magnetic field and adjust the magnetic force to perform steering to propel the microrobots 14 and 24 for delivery of the cell therapy product in an arbitrary 3D direction, thereby accurately moving the microrobots 14 and 24 for delivery of the cell therapy product to the affected area of the patient's body. The magnetic field generating device may be configured to include a plurality of electromagnetic coils.

FIGS. 7 and 8 are diagrams illustrating pathways of injecting the microrobots 14 and 24 for delivery of the cell therapy product into a patient's body according to an embodiment.

The microrobots 14 and 24 for delivery of the cell therapy product may be intranasally administered and moved to a diseased target site through a peri-olfactory pathway. Methods for administration through the peri-olfactory pathway may include the first pathway 3 and the second pathway 4.

Referring to FIG. 7, in the first pathway 3, an injection device may penetrate into the nasal cavity, and the microrobots 14 and 24 for delivery of the cell therapy product may be injected into the brain along the nasal cavity. The microrobots 14 and 24 for delivery for the cell therapy product injected through the first pathway 3 may be injected into the nasal cavity of the patient to bypass the blood brain barrier and subsequently delivered to the brain nervous system through an olfactory organ and a tertiary nervous system pathway. In addition, since the microrobots 14 and 24 may be injected into the nasal cavity, the cell therapy product may be administered to the brain through a minimal, non-invasive pathway. In addition, since the microrobots 14 and 24 for delivery of the cell therapy product may be administered and applied to a local site, side effects caused by exposure of the therapy product to the whole body may be reduced. In addition, the microrobots 14 and 24 may be precisely transferred to a target area with a disease.

Referring to FIG. 8, in the second pathway 4, the injection device may penetrate into the brain through the nasal cavity, and subsequently, the microrobots 14 and 24 for delivery of the cell therapy product may be injected directly into the brain. Since the injection device may be directly injected into the brain, the microrobots 14 and 24 for delivery of the cell therapy product may be more accurately transferred.

According to the embodiments, it is possible to provide the microrobots 14 and 24 for delivery of the cell therapy product and the fabrication method thereof, and to expect an equivalent or superior therapeutic effect to bone marrow-derived mesenchymal stem cells or adipose-derived mesenchymal stem cells having few side effects due to the same genetic origin as a subject to be administered using inferior turbinate-derived stem cells.

Furthermore, the microrobots 14 and 24 for delivery of the cell therapy product may increase the robot fabrication efficiency and promote the proliferation and differentiation in the affected area after transplantation by providing the microrobots 14 and 24 for delivery of the cell therapy product coated with the coating material 11 capable of promoting cell attachment, proliferation, and differentiation and the fabrication method thereof.

In addition, the microrobots 14 and 24 for delivery of the cell therapy product may be coated with the coating material 11 to increase the adhesive strength of the stem cells 13, thereby minimizing the loss amount of the stem cells 13 in the patient's body and enabling the treatment with low cost and high efficiency.

In addition, the microrobots 14 and 24 for delivery of the cell therapy product may be configured to be administered intranasally to efficiently deliver the cell therapy product to the brain nervous system through the olfactory organ and the tertiary nervous system pathway by bypassing the blood brain barrier, and to reduce side effects caused by exposure of the therapy product to the whole body.

In addition, it is possible to safely and precisely move the microrobots in the patient's body by providing the microrobots 14 and 24 for delivery of the cell therapy product capable of moving in the patient's body by a wireless control method using an external magnetic field and the fabrication method thereof.

As described above, although the embodiments have been described by the restricted drawings, various technical modifications and variations can be applied on the basis of the embodiments by one of ordinary skill in the art. For example, even if the described techniques are performed in a different order from the described method, and/or components such as a system, a structure, a device, a circuit, and the like described above are coupled or combined in a different form from the described method, or replaced or substituted by other components or equivalents, an appropriate result can be achieved.

Therefore, other implementations, other embodiments, and equivalents to the claim scope also belong to the scope of the following claims.

## Claims

1. A method for fabricating a microrobot for delivery of a cell therapy product, the method comprising:
coating a magnetic material with a coating material capable of promoting attachment, proliferation, and differentiation of stem cells; and
internalizing or attaching the coated magnetic material into stem cells.

2. The method of claim 1, wherein a single stem cell and the coated magnetic material become one microrobot.

3. The method of claim 1, further comprising:
culturing a plurality of stem cells in a spheroid shape, wherein the internalizing or attaching of the coated magnetic material comprises internalizing or attaching the coated magnetic material into the stem cells cultured in the spheroid shape.

4. The method of claim 3, wherein the culturing of the plurality of stem cells in the spheroid shape and the internalizing or attaching of the coated magnetic material into the stem cells comprise using at least one of a hanging-drop culture method or a U-shaped 96-well plate culture method.

5. The method of claim 1, wherein the stem cells are inferior turbinate-derived stem cells.

6. The method of claim 5, wherein the inferior turbinate-derived stem cells are differentiated into one of chondrocytes, bone cells, adipocytes, mucosal differentiation, and nerve cells.

7. A microrobot for delivery of a cell therapy product, the microrobot comprising:
stem cells;
a magnetic material attached to surfaces of the stem cells or injected into the stem cells to enable migration in response to an external magnetic field; and
a coating material coated on a surface of the magnetic material and promoting attachment, proliferation, and differentiation of the stem cells.

8. The microrobot of claim 7, wherein a single stem cell and the magnetic material coated with the coating material configure one microrobot.

9. The microrobot of claim 7, wherein a plurality of stem cells cultured in a spheroid shape and the magnetic material coated with the coating material configure one microrobot.

10. The microrobot of claim 7, wherein the microrobot is injected by any one of:
a first pathway in which an injection device penetrates into a nasal cavity, and the microrobot is injected into a brain along the nasal cavity; and
a second pathway in which the injection device penetrates into the brain through the nasal cavity, and the microrobot is injected directly into the brain.

11. The microrobot of claim 10, wherein the injection pathway of the microrobot further comprises a pathway in which the microrobot is also injected into affected areas with bone or cartilage disease, rheumatoid arthritis, degenerative nervous system disease, brain nervous system disease, and dementia disease.

12. The microrobot of claim 7, wherein the stem cells are able to be rotated and translated by an external magnetic field inside an organ by the magnetic material.

13. The microrobot of claim 7, wherein the stem cells are inferior turbinate-derived stem cells.

14. The microrobot of claim 13, wherein the inferior turbinate-derived stem cells are differentiated into one of chondrocytes, bone cells, adipocytes, mucosal differentiation, and nerve cells.
